# FASCICULE DE BREVET EUROPEEN

(11) **EP 3 952 770 B1**
(45) Date de publication et mention de la délivrance du brevet: **04.06.2025**
(21) Numéro de dépôt: 20717195.0
(22) Date de dépôt: 09.04.2020
(51) Int. Cl.: A61B 17/88, A61B 17/70, A61F 2/28, A61F 2/44

(54) **ENSEMBLE DE POSITIONNEMENT ET DE BLOCAGE EN POSITION D'UN BALLONNET GONFLABLE AU SEIN D'UN CORPS VERTEBRAL**
ANORDNUNG ZUR POSITIONIERUNG UND POSITIONSIMMOBILISIERUNG EINES AUFBLASBAREN BALLONS BEI EINEM WIRBELKÖRPER
ASSEMBLY FOR POSITIONING AND POSITIONALLY IMMOBILIZING AN INFLATABLE BALLOON WITHIN A VERTEBRAL BODY

(30) Priorité: 09.04.2019 FR 1903788
(43) Date de publication de la demande: 16.02.2022
(73) Titulaire: Safe Orthopaedics, 95610 Eragny-sur-Oise (FR)
(72) Inventeur: PETIT, Dominique, 62180 Verton (FR); VAN GAALEN, Steven, 3581 HR, Utrecht (NL)
(74) Mandataire: IP Trust
(86) Numéro de dépôt international: PCT/EP2020/060145
(87) Numéro de publication internationale: WO 2020/208135

(56) Documents cités:
- EP-A1- 2 730 246
- EP-A1- 3 081 178
- WO-A1-99/02214
- US-A1- 2004 092 946
- US-A1- 2009 299 378
- US-A1- 2010 274 080

## Description

### DOMAINE TECHNIQUE DE L'INVENTION

La présente invention concerne le domaine de la stabilisation des vertèbres, et plus particulièrement la restauration en hauteur (réduction) d'une vertèbre présentant un affaissement.

L'invention concerne plus particulièrement un ensemble de positionnement et de blocage en position d'un ballonnet gonflable au sein d'un corps vertébral, lequel ensemble comprend une pièce d'accès canulée allongée à une zone au sein du corps vertébral, un ballonnet gonflable monté en extrémité d'un cathéter apte à être raccordé à une unité de délivrance d'un fluide, l'ensemble ballonnet et cathéter étant arrangé pour traverser de manière coulissante la pièce d'accès.

Par cathéter, on entend dans la présente demande un tube mince et flexible permettant l'injection et/ou l'évacuation d'un liquide.

### ETAT DE LA TECHNIQUE

Parmi les techniques de réduction d'une vertèbre affaissée, on connait la kyphoplastie. Cette technique consiste à réaliser la restauration de la hauteur vertébrale à l'aide d'un ballonnet, ou de tout autre dispositif apte à être expansé au sein du corps vertébral, placé dans le corps vertébral avant l'injection de ciment. Le ballonnet, et plus généralement le dispositif, est inséré par voie percutanée à travers le pédicule. A l'aide d'un cathéter, d'une seringue ou similaire, à l'extrémité duquel il est monté, le ballonnet est guidé au travers d'une canule de travail jusqu'au corps vertébral pour être ensuite gonflé de manière à réduire la fracture. Après réduction de la fracture, le ballonnet est dégonflé puis retiré. La cavité résultante reçoit alors un dépôt de ciment osseux pour stabiliser la fracture. WO99/02214 A1 décrit un appareil pour réparer un espace effondré à l'intérieur des corps vertébraux, qui comprend un introducteur avec un ancrage fileté qui facilite le positionnement, la fixation ou le montage de l'introducteur sur le corps vertébral.

EP 3 081 178 A1 décrit un système d'implantation d'un produit de comblement osseux, comprenant un ballonet attaché a un cathéter.

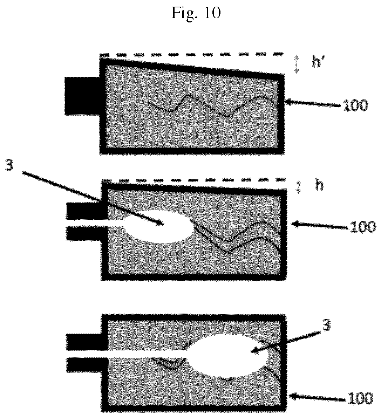

L'une des problématiques rencontrées avec cette technique est le positionnement du ballonnet au sein de la vertèbre à restaurer. Un positionnement trop ou au contraire insuffisamment avancé au sein de la vertèbre a une influence sur le redressement de la vertèbre comme le montre la figure 10. Ainsi, et comme illustré sur la figure 10b, un ballonnet 3 insuffisamment avancé au sein de la vertèbre 100 ne permet pas de restaurer totalement la hauteur de la vertèbre : on peut voir en effet un décalage entre la hauteur de la vertèbre redressée et la hauteur optimale de redressement. Au contraire, comme illustré sur la figure 10c, le positionnement d'un ballonnet 3 plus loin dans le corps vertébral permet un redressement optimal de la vertèbre 100.

Une autre problématique rencontrée avec cette technique réside dans le maintien du ballonnet au sein de la vertèbre à redresser. En effet, sous l'action des efforts exercés sur le ballonnet par la vertèbre lors de son redressement, le ballonnet a tendance à glisser vers la zone dans laquelle s'exerce le moins de contraintes, de sorte que l'effet du ballonnet sur la restauration en hauteur de la vertèbre peut être amoindri. Le redressement en hauteur de la vertèbre sera d'autant moins important que le ballonnet sera peu avancé dans le corps vertébral.

L'invention vise à remédier à ces problèmes en proposant un ensemble offrant un positionnement stabilisé du ballonnet gonflable au sein du corps vertébral pour ainsi assurer un redressement optimal de la vertèbre affaissée et ce, quel que soit le degré d'affaissement de cette dernière.

### OBJET DE L'INVENTION

A cet effet, et selon un premier aspect, l'invention propose un ensemble de positionnement et de blocage en position d'un ballonnet gonflable au sein d'un corps vertébral, comprenant une pièce d'accès au corps vertébral, canulée et de forme allongée, un ballonnet gonflable monté en extrémité d'un cathéter apte à être raccordé à une unité de délivrance d'un fluide, l'ensemble ballonnet et tige étant arrangé pour traverser de manière coulissante la pièce d'accès, l'ensemble étant remarquable en ce que la pièce d'accès comporte des moyens d'ancrage dans le corps vertébral et en ce qu'il comporte en outre des moyens de blocage en position du cathéter pourvu du ballonnet sur la pièce d'accès.

La pièce d'accès, du fait de ses moyens d'ancrage dans le corps vertébral, forme, une fois ancrée dans la vertèbre, un canal de travail stabilisé par rapport à la vertèbre tandis que les moyens de blocage permettent de bloquer tout mouvement de coulissement du cathéter au travers de la pièce d'accès. La présence des moyens de blocage combiné avec l'ancrage de la pièce d'accès permet ainsi à la pièce d'accès ancrée d'établir un point fixe par rapport à la vertèbre mais aussi par rapport au cathéter. Cela facilite ainsi la mise en place du ballonnet et assure le maintien en position du celui-ci au sein de la vertèbre. Le positionnement du ballonnet n'est donc plus aléatoire comme cela est le cas avec les systèmes actuels de kyphoplastie, tout glissement du ballonnet lors de son gonflement étant évité.

Avantageusement, les moyens d'ancrage consistent en au moins un filet.

Avantageusement, la pièce d'accès est un implant.

Selon une variante de réalisation, la pièce d'accès comprend un instrument prolongé de l'implant.

Avantageusement, l'implant est une cheville d'ancrage.

Selon une autre variante, la pièce d'accès est une canule. Avantageusement, la canule est configurée pour être traversée par un instrument de forage monté coulissant, ledit instrument de forage comportant un axe pourvu d'une extrémité distale effilée de sorte à permettre le forage du corps vertébral, la canule et l'axe de forage étant dimensionnés l'un par rapport à l'autre pour que l'extrémité distale effilée de la tige de forage s'étende au-delà de la canule.

Avantageusement, les moyens de blocage sont arrangés pour autoriser un réglage du positionnement du cathéter par rapport à la pièce d'accès et bloquer le cathéter dans la position choisie par rapport à ladite pièce.

Avantageusement, les moyens de blocage comportent un manchon monté coulissant sur le cathéter et des moyens de solidarisation du manchon sur la pièce d'accès.

Avantageusement, le manchon comporte une fente de montage longitudinale pour le passage du cathéter au travers dudit manchon, ladite fente étant arrangée pour maintenir le manchon sur le cathéter tout en permettant son coulissement le long de ce dernier.

### BREVE DESCRIPTION DES FIGURES

D'autres caractéristiques et avantages de l'invention ressortiront de la description détaillée de l'invention qui va suivre en référence aux figures annexées et dans lesquelles :
La figure 1 représente un ensemble de positionnement et de blocage en position d'un ballonnet gonflable au sein d'un corps vertébral selon un premier exemple de réalisation ;
La figure 2 représente l'ensemble de la figure 1 en position dans une vertèbre, le ballonnet représenté en position dans le corps vertébral ;
La figure 3 représente une vue de détail de la partie proximale de l'ensemble de la figure 1 assurant le blocage du ballonnet ;
La figure 4 représente une vue de détail de la partie distale de l'ensemble de la figure 1 assurant le blocage du ballonnet ;
La figure 5 représente un ensemble de positionnement et de blocage en position d'un ballonnet gonflable au sein d'un corps vertébral selon un deuxième exemple de réalisation ;
La figure 6 représente l'ensemble de la figure 5 en position dans une vertèbre, le ballonnet représenté en position dans le corps vertébral ;
La figure 7 représente une vue de détail de la partie proximale de l'ensemble de la figure 5 assurant le blocage du ballonnet ;
La figure 8 représente une vue de détail de la partie distale de l'ensemble de la figure 5 assurant le blocage du ballonnet ;
La figure 9 représente une vue de détail des moyens de blocage du ballonnet ;
La figure 10 montre l'impact du positionnement du ballonnet au sein d'une vertèbre sur le redressement de cette dernière.

Pour plus de clarté, les éléments identiques ou similaires des différents modes de réalisation sont repérés par des signes de référence identiques sur l'ensemble des figures.

### DESCRIPTION DETAILLEE DE L'INVENTION

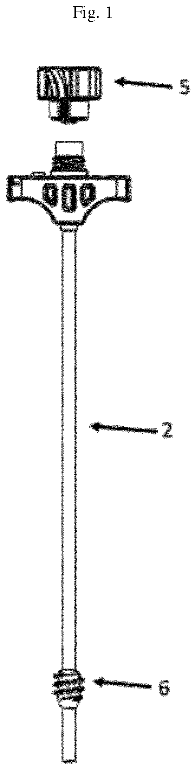

En relation avec les figures 1 à 4, il est décrit un exemple de réalisation d'un ensemble 1 permettant le positionnement et le blocage en position d'un ballonnet 3 gonflable au sein d'un corps vertébral.

L'ensemble 1 comprend deux sous-ensembles : un premier sous-ensemble comprenant une pièce d'accès dans le corps vertébral, ladite pièce-étant de forme allongée et canulée, et un deuxième sous-ensemble formé d'un ballonnet 3 gonflable monté en extrémité d'un cathéter 4 avantageusement flexible. Le sous ensemble ballonnet / tige (cathéter) est arrangé pour coulisser au travers de la pièce d'accès 2. Dans l'exemple illustré, la pièce d'accès est une canule rigide. Avantageusement, le cathéter 4 comporte des moyens de raccordement 40 à une unité de délivrance d'un fluide.

Selon l'invention, l'ensemble 1 comporte des moyens assurant la stabilisation du ballonnet 3 par rapport au corps vertébral. Les moyens de stabilisation du ballonnet 3 comportent des premiers moyens assurant le blocage en position du cathéter, portant en extrémité le ballonnet 3, sur la pièce d'accès 2 et des deuxièmes moyens assurant l'ancrage de la pièce d'accès 2 dans le corps vertébral.

Avantageusement, les moyens de blocage se présentent sous la forme d'un manchon 5 monté coulissant de long du cathéter 4.

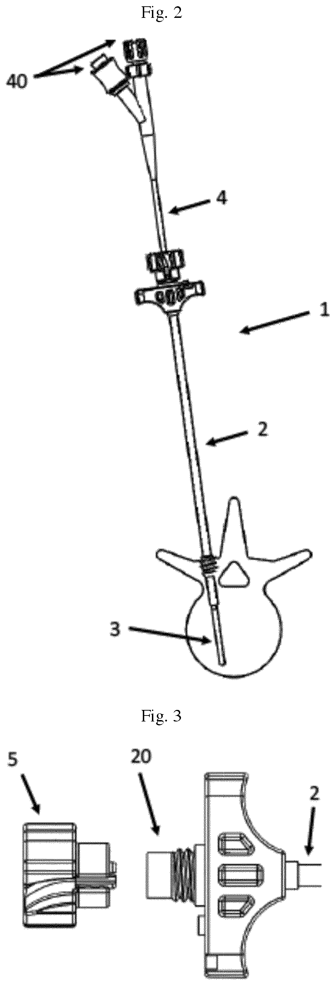

Selon un exemple de réalisation particulier illustré sur la figure 3, le manchon 5 et la pièce d'accès 2 comportent des moyens d'association réciproque. Dans le mode de réalisation illustré sur la figure 3, le manchon 5 est vissé sur un embout d'accroche 20 ménagé en extrémité proximale de la pièce d'accès 2.

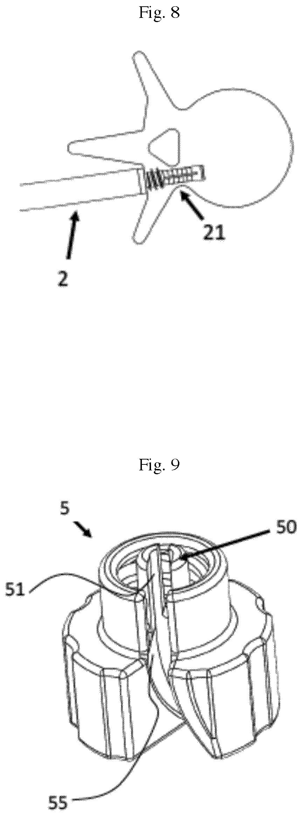

Dans le mode de réalisation décrit, le manchon 5 comporte une fente de montage 55 longitudinale permettant le passage du cathéter 4 (figure 9). La fente 55 est arrangée pour maintenir le manchon 5 sur le cathéter tout en autorisant son coulissement le long de celui-ci.

Le manchon 5 comporte également un embout de retenue 50 du cathéter 4 lorsqu'il est en place sur la pièce d'accès 2. L'embout de retenue 50 est un embout déformable de type pince. Il présente une paroi latérale pourvue d'une fente de passage s'étendant sur tout sa longueur et communiquant avec la fente de montage 55. L'embout de retenue 50 est arrangé pour s'insérer à l'intérieur de l'embout d'accroche 20 de la pièce d'accès 2. L'embout de retenue 50 présente une paroi interne tronconique dimensionnée pour que, lors de son insertion dans l'embout d'accroche 20 de la pièce d'accès 2, sa section se rétrécisse au contact de la paroi interne de l'embout d'accroche 20 de sorte à enserrer et bloquer le mouvement du cathéter 4 du ballonnet 3.

Les moyens d'ancrage consistent, dans l'exemple illustré, à un filet 6 prévu sur la paroi périphérique de la pièce canulée 2.

Avantageusement, la canule 2 est configurée pour être traversée par un instrument de forage monté coulissant dans celle-ci et comportant un axe pourvu d'une extrémité distale effilée permettant le forage du corps vertébral. La canule et l'axe de forage sont dimensionnés l'un par rapport à l'autre pour que l'extrémité distale effilée de l'axe de forage s'étende au-delà de la canule. La canule et l'instrument de forage forment ainsi avantageusement un instrument de type Jamshidi^{™}.

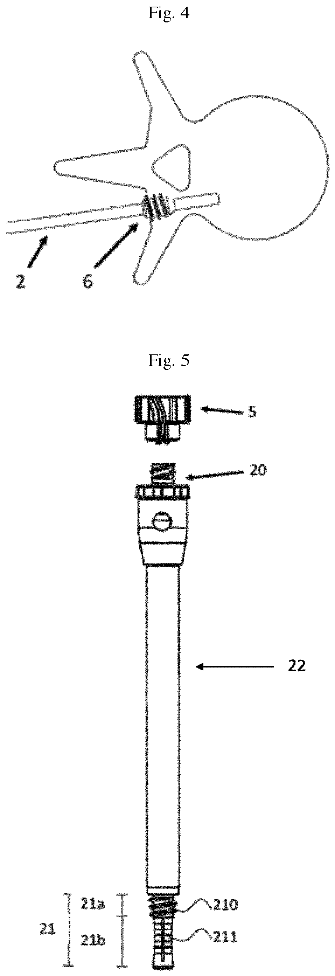

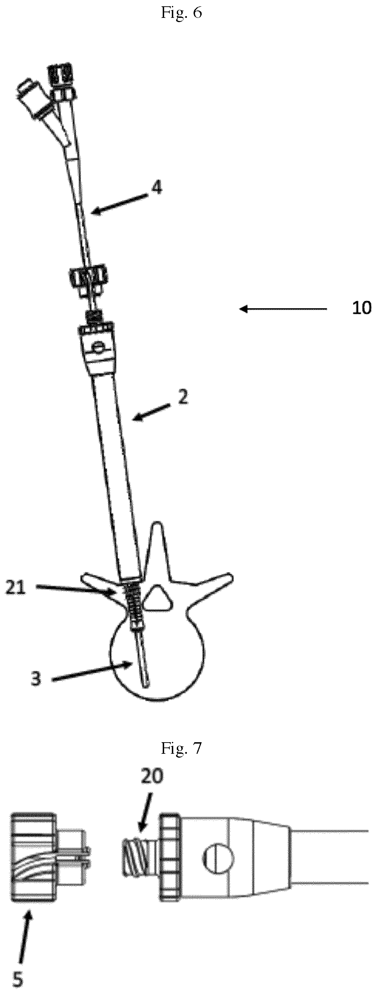

Les figures 5 à 8 illustrent un autre exemple de réalisation d'un ensemble 10 permettant le positionnement et le blocage en position d'un ballonnet 3 gonflable. Dans ce mode de réalisation, l'ensemble 10 de positionnement et de blocage en position du ballonnet reprend l'ensemble des caractéristiques de l'ensemble précédemment décrit, et notamment la partie blocage du ballonnet 3 par rapport à la pièce d'accès. Il diffère cependant par la constitution de la pièce d'accès 2 et les moyens d'ancrage mis en œuvre. Selon cet exemple de réalisation, la pièce d'accès 2 est formée non pas par une canule comme dans l'exemple précédemment décrit, mais par un instrument couplé à un implant. Plus particulièrement, et comme illustré sur la figure 5, la pièce d'accès 2 comprend un tube 22 pourvu en extrémité proximale d'un implant canulé 21. Dans l'exemple illustré, l'implant 21 est une cheville d'ancrage.

La cheville d'ancrage 21, dans l'exemple illustré, se présente sous la forme d'un corps de cheville cylindrique canulé comprenant deux parties : une partie supérieure 21a pourvue d'un filetage 210 hélicoïdal externe et une partie inférieure 21b expansible. La cheville 21 peut être réalisée en un matériau thermoplastique, de préférence en PEEK, ou en métal. La partie supérieure 210 pourvue du filetage permet un ancrage primaire de la cheville dans le corps vertébral. Elle définit une zone s'étendant approximativement sur un tiers de la longueur de la cheville 4. La partie inférieure 21b comporte des fentes longitudinales délimitant des bras pouvant s'expanser radialement. Les fentes longitudinales sont avantageusement non débouchantes en extrémité proximale de la cheville. Les bras sont ainsi reliés entre eux en extrémité proximale. La partie inférieure 21b comporte également des crans radiaux 211 externes arrangés pour bloquer le mouvement de la cheville dans une direction opposée à celle de son insertion dans le corps vertébral, et empêchant ainsi tout retrait intempestif de la cheville lorsque celle-ci est ancrée dans le corps vertébral.

Comme on le comprend, la cheville 21 constitue les moyens d'ancrage au corps vertébral.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Ensemble (1, 10) de positionnement et de blocage en position d'un ballonnet (3) gonflable au sein d'un corps vertébral, comprenant une pièce d'accès (2) au corps vertébral, canulée et de forme allongée, et un ballonnet (3) gonflable monté en extrémité d'un cathéter (4) apte à être raccordé à une unité de délivrance d'un fluide, l'ensemble ballonnet (3) et cathéter étant arrangé pour traverser de manière coulissante la pièce d'accès (2), la pièce d'accès (2) comporte des moyens d'ancrage dans le corps vertébral, **caractérisé en ce que** l'ensemble comprend en outre des moyens de blocage en position du cathéter pourvu du ballonnet (3) sur la pièce d'accès (2).

2. Ensemble (1, 10) de positionnement et de blocage en position d'un ballonnet (3) gonflable selon la revendication 1, **caractérisé en ce que** les moyens d'ancrage consistent en au moins un filet (6).

3. Ensemble (1, 10) de positionnement et de blocage en position d'un ballonnet (3) gonflable selon la revendication 1 ou la revendication 2, **caractérisé en ce que** la pièce d'accès (2) est un implant.

4. Ensemble (1, 10) de positionnement et de blocage en position d'un ballonnet (3) gonflable selon la revendication 1 ou la revendication 2, **caractérisé en ce que** la pièce d'accès (2) comprend un instrument prolongé d'un implant.

5. Ensemble (1, 10) de positionnement et de blocage en position d'un ballonnet (3) gonflable selon la revendication 3 ou la revendication 4, **caractérisé en ce que** l'implant est une cheville d'ancrage (21).

6. Ensemble (1, 10) de positionnement et de blocage en position d'un ballonnet (3) gonflable selon la revendication 1 ou la revendication 2, **caractérisé en ce que** la pièce d'accès (2) est une canule.

7. Ensemble (1, 10) de positionnement et de blocage en position d'un ballonnet (3) gonflable selon la revendication 6, **caractérisé en ce que** la canule est configurée pour être traversée par un instrument de forage monté coulissant, ledit instrument de forage comportant un axe pourvu d'une extrémité distale effilée de sorte à permettre le forage du corps vertébral, la canule et l'axe de forage étant dimensionnés l'un par rapport à l'autre pour que l'extrémité distale effilée de l'axe de forage s'étende au-delà de la canule.

8. Ensemble (1, 10) de positionnement et de blocage en position d'un ballonnet (3) gonflable selon l'un quelconque des revendications précédentes, **caractérisé en ce que** les moyens de blocage sont arrangés pour autoriser un réglage du positionnement du cathéter par rapport à la pièce d'accès (2) et bloquer le cathéter dans la position choisie par rapport à ladite pièce.

9. Ensemble (1, 10) de positionnement et de blocage en position d'un ballonnet (3) gonflable selon l'une quelconque des revendications précédentes, **caractérisé en ce que** les moyens de blocage comportent un manchon (5) monté coulissant sur le cathéter (4) et des moyens de solidarisation du manchon sur la pièce d'accès (2).

10. Ensemble (1, 10) de positionnement et de blocage en position d'un ballonnet (3) gonflable selon la revendication 9, **caractérisé en ce que** le manchon (5) comporte une fente de montage (55) longitudinale pour le passage du cathéter au travers dudit manchon, ladite fente de montage (55) étant arrangée pour maintenir le manchon (5) sur le cathéter tout en permettant son coulissement le long de ce dernier.

## Patentansprüche

1. Anordnung (1, 10) zum Positionieren und Blockieren eines aufblasbaren Ballons (3) in einer Position innerhalb eines Wirbelkörpers, umfassend ein Zugangsteil (2) zu dem Wirbelkörper, das mit einer Kanüle versehen ist und eine längliche Form besitzt, und einen aufblasbaren Ballon (3), der an einem Ende eines Katheters (4) befestigt ist, der geeignet ist, an eine Fluidzufuhreinheit angeschlossen zu werden, wobei die Anordnung aus Ballon (3) und Katheter eingerichtet ist, um das Zugangsteil (2) gleitend zu durchqueren, wobei das Zugangsteil (2) Verankerungsmittel in dem Wirbelkörper aufweist,
**dadurch gekennzeichnet, dass** die Anordnung ferner Mittel zum Blockieren des mit dem Ballon (3) versehenen Katheters in der Position auf dem Zugangsteil (2) umfasst.

2. Anordnung (1, 10) zum Positionieren und Blockieren eines aufblasbaren Ballons (3) in der Position nach Anspruch 1, **dadurch gekennzeichnet, dass** die Verankerungsmittel aus mindestens einem Gewindegang (6) bestehen.

3. Anordnung (1, 10) zum Positionieren und Blockieren eines aufblasbaren Ballons (3) in der Position nach Anspruch 1 oder 2,
**dadurch gekennzeichnet, dass** das Zugangsteil (2) ein Implantat ist.

4. Anordnung (1, 10) zum Positionieren und Blockieren eines aufblasbaren Ballons (3) in der Position nach Anspruch 1 oder 2,
**dadurch gekennzeichnet, dass** das Zugangsteil (2) ein mit einem Implantat verlängertes Instrument umfasst.

5. Anordnung (1, 10) zum Positionieren und Blockieren eines aufblasbaren Ballons (3) in der Position nach Anspruch 3 oder 4,
**dadurch gekennzeichnet, dass** das Implantat ein Verankerungsstift (21) ist.

6. Anordnung (1, 10) zum Positionieren und Blockieren eines aufblasbaren Ballons (3) in der Position nach Anspruch 1 oder 2,
**dadurch gekennzeichnet, dass** das Zugangsteil (2) eine Kanüle ist.

7. Anordnung (1, 10) zum Positionieren und Blockieren eines aufblasbaren Ballons (3) in der Position nach Anspruch 6, **dadurch gekennzeichnet, dass** die Kanüle konfiguriert ist, um von einem gleitend befestigten Bohrinstrument durchquert zu werden, wobei das Bohrinstrument eine Achse, die mit einem verjüngten distalen Ende versehen ist, aufweist, um das Bohren des Wirbelkörpers zu ermöglichen, wobei die Kanüle und die Bohrachse relativ zueinander bemessen sind, sodass das verjüngte distale Ende der Bohrachse sich über die Kanüle hinaus erstreckt.

8. Anordnung (1, 10) zum Positionieren und Blockieren eines aufblasbaren Ballons (3) in der Position nach einem der vorstehenden Ansprüche,
**dadurch gekennzeichnet, dass** die Blockiermittel eingerichtet sind, um eine Einstellung der Positionierung des Katheters relativ zu dem Zugangsteil (2) zuzulassen und den Katheter in der gewählten Position relativ zu dem Teil zu blockieren.

9. Anordnung (1, 10) zum Positionieren und Blockieren eines aufblasbaren Ballons (3) in der Position nach einem der vorstehenden Ansprüche,
**dadurch gekennzeichnet, dass** die Blockiermittel eine auf dem Katheter (4) gleitend befestigte Hülse (5) und Mittel zum festen Verbinden der Hülse an dem Zugangsteil (2) aufweisen.

10. Anordnung (1, 10) zum Positionieren und Blockieren eines aufblasbaren Ballons (3) in der Position nach Anspruch 9, **dadurch gekennzeichnet, dass** die Hülse (5) einen längsverlaufenden Befestigungsschlitz (55) für die Durchführung des Katheters durch die Hülse aufweist, wobei der Befestigungsschlitz (55) eingerichtet ist, um die Hülse (5) auf dem Katheter zu halten und ihr gleichzeitig zu ermöglichen, entlang dieses Letzteren zu gleiten.

## Claims

1. Assembly (1, 10) for positioning and locking an inflatable balloon (3) in position within a vertebral body, comprising an access piece (2) to the vertebral body, which piece is cannulated and elongate, and an inflatable balloon (3) mounted at the end of a catheter (4) suitable for being connected to a fluid delivery unit, the balloon (3) and catheter assembly being arranged to slide through the access piece (2), the access piece (2) comprises means for anchoring in the vertebral body, **characterized in that** the assembly further comprises means for locking the catheter provided with the balloon (3) in position on the access piece (2).

2. Assembly (1, 10) for positioning and locking an inflatable balloon (3) in position according to claim 1, **characterized in that** the anchoring means consist of at least one thread (6).

3. Assembly (1, 10) for positioning and locking an inflatable balloon (3) in position according to either claim 1 or claim 2, **characterized in that** the access piece (2) is an implant.

4. Assembly (1, 10) for positioning and locking an inflatable balloon (3) in position according to either claim 1 or claim 2, **characterized in that** the access piece (2) comprises an instrument extended by an implant.

5. Assembly (1, 10) for positioning and locking an inflatable balloon (3) in position according to either claim 3 or claim 4, **characterized in that** the implant is an anchoring pin (21).

6. Assembly (1, 10) for positioning and locking an inflatable balloon (3) in position according to either claim 1 or claim 2, **characterized in that** the access piece (2) is a cannula.

7. Assembly (1, 10) for positioning and locking an inflatable balloon (3) in position according to claim 6, **characterized in that** the cannula is configured to be passed through by a slidably mounted boring instrument, said boring instrument comprising a shaft provided with a distal end that is tapered so as to allow boring of the vertebral body, the cannula and the boring shaft being dimensioned relative to one another so that the tapered distal end of the boring shaft extends beyond the cannula.

8. Assembly (1, 10) for positioning and locking an inflatable balloon (3) in position according to any of the preceding claims, **characterized in that** the locking means are arranged to allow adjustment of the positioning of the catheter relative to the access piece (2) and to lock the catheter in the chosen position relative to said piece.

9. Assembly (1, 10) for positioning and locking an inflatable balloon (3) in position according to any of the preceding claims, **characterized in that** the locking means comprise a sleeve (5) slidably mounted on the catheter (4) and means for securing the sleeve to the access piece (2).

10. Assembly (1, 10) for positioning and locking an inflatable balloon (3) in position according to claim 9, **characterized in that** the sleeve (5) comprises a longitudinal mounting slot (55) for the passage of the catheter through said sleeve, said mounting slot (55) being arranged to hold the sleeve (5) on the catheter while allowing it to slide along the latter.
